# EUROPEAN PATENT APPLICATION

(11) **EP 1 192 928 A2**
(43) Date of publication of application: **03.04.2002**
(21) Application number: 01500227.2
(22) Date of filing: 11.09.2001
(51) Int. Cl.: A61J 17/00

(54) **Comforter adaptable to the tongue and palate**

(30) Priority: 25.09.2000 ES 200002307
(71) Applicant: Alba Quiros de, Miguel, 08029 Barcelona (ES)
(72) Inventor: Alba Quiros de, Miguel, 08029 Barcelona (ES)
(74) Representative: Renter Llenas, Maria

(57) **Abstract**

The suction device is made up of a U shaped body (5) whose arms contain horizontal cavities to be engaged and centered with the teeth (15), and a bulb shaped section, extending from the internal side of the U shaped body (5) and being attached to this latter by means of a narrow shaped neck, expanding to form a flat base which is adaptable to the tongue and a curved upper portion adaptable to the palate, being said bulb shaped section, both flexible and adaptable and having orifices through which air may pass, which orifices communicate with one of the cavities in the U shaped part of the structure, to allow for the passage of the air in accordance with the suction cycles. The device permits the recovery of the tone of the jaw muscles and the soft palate and avoids and corrects the ogival palate, permitting as well as to increase ventilation space in the rear area of the nasal cavities.

## Description

This invention consists of a suction device adaptable to the tongue and palate, which regulates the tone of the jaw muscles and the soft palate and, likewise, avoids or corrects the ogival palate, as well as helping with the correct positioning of the molar bones which, in turn, allows for an increased ventilation space in the rear area of the nasal cavities. This also prevents the adenoids from obstructing the air flow. Another of the practical functions of this suction device is that it avoids apnea during sleep.

For its correct functioning, this invention consists of a device made up of a body to be centered in the dental arc, with a U or horse shoe shaped structure adapted to the shape of the dental arc, with lower and upper cavities designed to align themselves correspondingly with the teeth. This structure is combined with a bulb shaped suction element, which extends along the inside edge of the horseshoe shaped device. Its bulb shaped body forms a substantially curved structure on its upper side to fit the palate, and its lower edge, which is comparatively flat, is designed to fit over the user's tongue, providing an opening, or openings, through which air may pass.

The suction element of this device helps to avoid:
- The wearing down of teeth due to bruxism
- Slackness in the soft palate, corrected by the suction tongue which tones up the palate, thereby avoiding snoring which slackness produces;
- Nocturnal apnea, caused by the obstruction of the respiratory track due to the collapse of the soft palate and the uvula;
- Night time tension of the jaw muscles and tension in the scalp produced whilst sleeping.

The device also achieves the following significant results:
- To normalise the development of the oral stage in the adult.
- Correction of the ogival palate, by adjusting dental occlusion. (For orthodontic use).
- Assistance in cases of emotional tension and stress at night.
- Perfect alignment of the molar bones achieved through the correction of the ogival palate, which, in turn, corrects visual disturbances, such as double vision, endophoria, exophoria and excess tension in the sclera.
- Realignment of the molar bones allows for an increase in the ventilation space of the rear opening of the nasal cavities, whereby the adenoids cannot obstruct the air flow.

This invention is explained below in more detail, making reference to the attached drawings, in which:

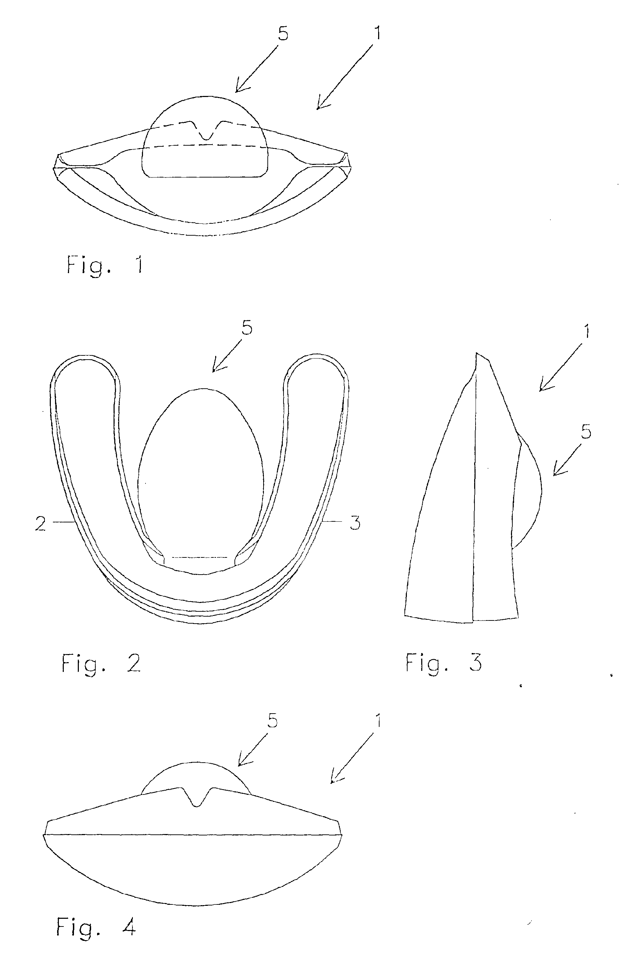

Figure 1 shows an elevated frontal view of the device.
Figure 2 shows a top view.
Figure 3 shows an elevated side view.
Figure 4 shows an elevated rear view.
Figure 5 shows a cross section, demonstrating the position of the device in the mouth.
Figure 6 shows a schematic top view of the positioning of the device in the mouth.
Figures 7 and 8 each show detailed sections of the device from the top.

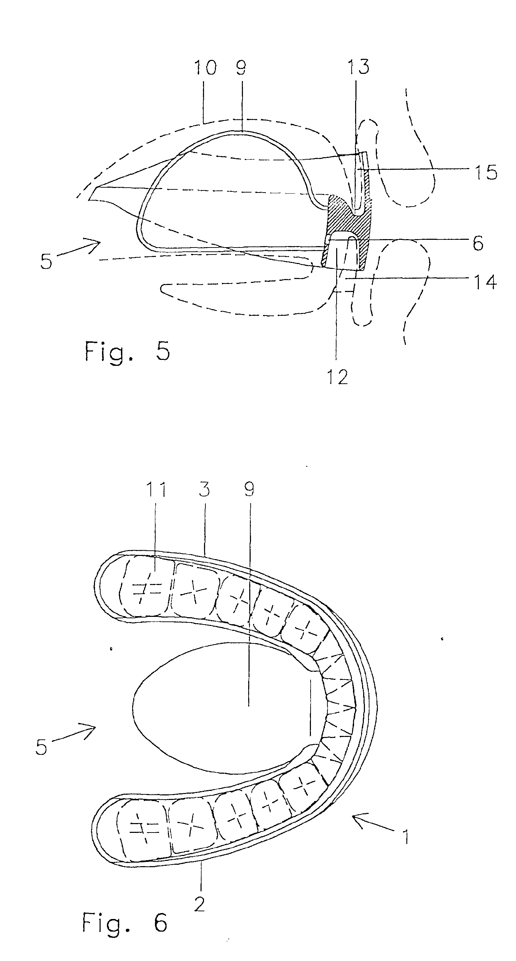

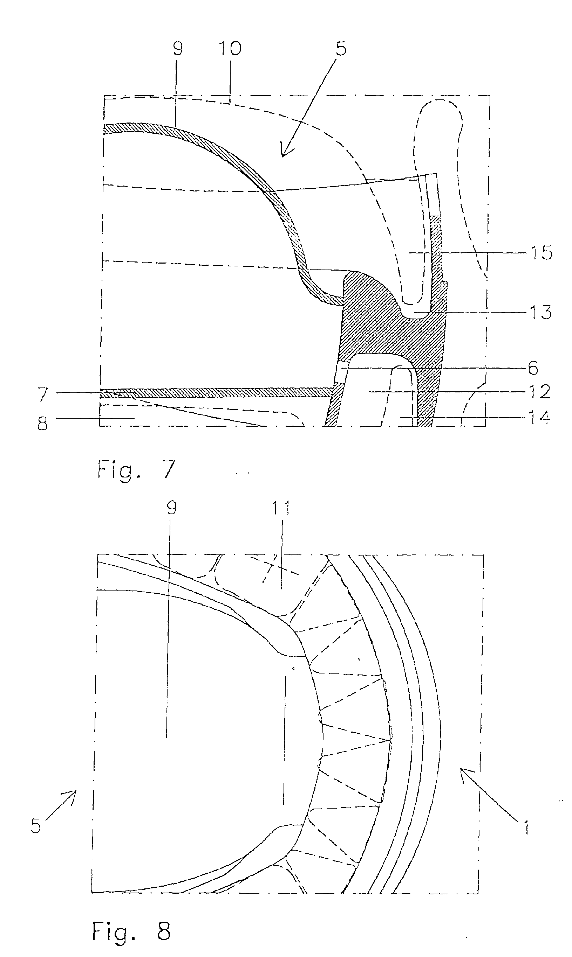

The suction device of this invention is made up of, as illustrated in the drawings, a centering and retention body -1- in a U or horse shoe shape, with arms -2- and -3- substantially adapted to the form of the dental arc. This horse shoe shaped body for centering and retention includes, attached to the inside edge by means of a narrow neck, an elastic, flexible, hollow bulb shaped section -4-, with one or more openings -6-, figures 5 and 7, in order to allow breathing during the suction cycles. This bulb shaped body -5- consists of an essentially flat base -7-, designed to be adapted to the user's tongue -8and an upper, curved section adaptable to the arch of the palate -10-.

The arms -2- and -3- of the body -1- comprise lengthwise cavities designed to fit the shape of the teeth outlined in figure 6 and are indicated by the number -11-. These cavities are represented by the numbers -12- and -13- in figures 5 and 7 in which the air openings -6- can also be observed, as well as the teeth -14- and -15-, which are represented in dashed lines corresponding respectively to the lower and upper jawbones.

Preferably, the parts of the suction device of this invention may be either manufactured in the same entirely flexible and adaptable material, such as latex, silicone or others or using different materials or a combination of hard or semi hard plastic for the centering 'U' shaped body, combined with a suitable adaptable and flexible material for the suction device, so that it may retain its shape after each suction cycle. The device should be manufactured in various sizes in order to be adaptable to different mouth shapes and age groups.

The device of this invention will permit the introduction of modifications, with the sole limitation of the appended claims.

## Claims

1. Suction device adaptable to the tongue and palate, **characterized in that** it is made up of a U shaped body whose arms contain horizontal cavities to be engaged and centered with the teeth, and a bulb shaped section, extending from the internal side of the U shaped body and being attached to this latter by means of a narrow shaped neck, expanding to form a flat base which is adaptable to the tongue and a curved upper portion adaptable to the palate, being said bulb shaped section, both flexible and adaptable and having orifices through which air may pass, which orifices communicate with one of the cavities in the U shaped part of the structure, to allow for the passage of the air in accordance with the suction cycles.

2. Device, according to claim 1, **characterized in that** the U shaped centering and retention body is made up of a flexible material of the same characteristics as the bulb shaped body adaptable to the tongue and palate.

3. Device, according to claim 1, **characterized in that** the U shaped centering and retention body is made up of a semi rigid material.
